**Europäisches Patentamt**

**European Patent Office**

**Office européen des brevets**

(11) Veröffentlichungsnummer: **0 385 281**
**A2**

## EUROPÄISCHE PATENTANMELDUNG

(21) Anmeldenummer: **90103486.8**

(22) Anmeldetag: **23.02.90**

(51) Int. Cl.5: **G01N 23/12, G01N 33/44**

(30) Priorität: **28.02.89 DE 3906203**

(43) Veröffentlichungstag der Anmeldung:
**05.09.90 Patentblatt 90/36**

(84) Benannte Vertragsstaaten:
**BE DE ES FR GB IT NL**

(71) Anmelder: **HOECHST AKTIENGESELLSCHAFT**
**Postfach 80 03 20**
**D-6230 Frankfurt am Main 80(DE)**

(71) Anmelder: **LÖFFEL VERFAHRENSTECHNIK GMBH**
**Greschbach-Strasse 33**
**D-7500 Karlsruhe(DE)**

(72) Erfinder: **Fritz, Hans-Gerhard, Prof.-Dr.**
**Gotenweg 10**
**D-7336 Uhingen(DE)**
Erfinder: **Löffel, Rudi**
**Max-Beckmann-Strasse 33**
**D-7500 Karlsruhe(DE)**

(54) Verfahren und Vorrichtung zur prozesskonformen Erfassung von Mischungsverhältnissen bei der Kunststoff- und Kautschukaufbereitung.

(57) Es wurde ein Verfahren und eine Vorrichtung gefunden, mit deren Hilfe während der Extrusion von Polymerschmelzen wichtige Eigenschaften ermittelt werden können:
- Kontinuierliche Messung der Dichte reiner Polymerer (Dichtekonstanz)
- Aufnahme von p,v,T-Diagrammen von Kunststoffen
- Ermittlung von Volumen- und Gewichtsanteilen bei Polymer/Füllstoff- bzw. Polymer/Verstärkungsmittel-Gemischen
- Ermittlung von Gewichtsanteilen Pigment bei der Masterbatch-Herstellung
- Ermittlung von Meßdaten für weiterführende Aussagen hinsichtlich mischtechnischer und thermischer Homogenität des Werkstoffs.
Ferner eignet sich die Vorrichtung als Sensor als Grundbaustein für einen geschlossenen Regelkreis zur Überwachung und Konstanthaltung von Füllstoffanteilen.

EP 0 385 281 A2

**Fig. 1**

## Verfahren und Vorrichtung zur prozeßzeitkonformen Erfassung von Mischungsverhältnissen bei der Kunststoff- und Kautschukaufbereitung

Die Erfindung bezieht sich auf ein Verfahren und eine Vorrichtung zur prozeßzeitkonformen Erfassung und Quantifizierung von Polymer/Polymer-, Polymer/Füllstoff-, Polymer/Verstärkungsstoff-, Polymer/Pigment-, Polymer/Keramik- und Polymer/Metallpulver-Mischungsverhältnissen bei Materialaufbereitungsprozessen mittels einer In-line-Meßtechnik.

Bei der Verarbeitung von Kunststoffen und Elastomeren werden in zunehmendem Maße sehr unterschiedlich komponierte Polymer/Zuschlagstoff-Gemische eingesetzt. Beispielhaft sind zu nennen: Mit mineralischen Füllstoffen (Talkum, Kreide, Kieselerde) versetzte Thermoplaste, mit faserförmigen Verstärkungsstoffen (Glas-, Kohlenstoffasern) modifizierte Polymere, mit Farbstoffen und Pigmenten ($Cr_2O_3$, $TiO_2$, $Fe_2O_3$) versetzte polymere Matrixwerkstoffe (sogenannte Masterbatches) und nicht zuletzt sogenannte Polyblends, die durch Mischen von zwei oder drei Thermoplast- oder Elastomertypen entstehen. Die Herstellung derartiger Gemische erfolgt im allgemeinen auf ein- oder zweiwelligen Schneckenpressen, deren Schnecken aufgabenspezifisch zahlreiche Misch- und Scherteile enthalten. Die Zuführung der Gemischkomponenten in das Aufbereitungsaggregat wird mittels volumetrisch oder gravimetrisch arbeitenden Dosiersystemen realisiert. Das im Extruder aufbereitete Gemisch wird unter Verwendung einer direkt mit der Schneckenpresse gekoppelten Granulierstation in zylinder-, linsen-, würfel- oder kugelförmige Granulate überführt, welche dann in Verarbeitungsanlagen, wie beispielsweise Spritzgießmaschinen, Hohlkörperblasanlagen oder Extrusionslinien, zu Fertigprodukten oder Halbzeugen geformt werden. In zunehmendem Maße werden derartige Aufbereitungs- und Formgebungsschritte auch zu Einstufenprozessen verknüpft.

Die Durchsatzleistungen bei solchen Gemischaufbereitungsprozessen liegen mit 500 kg/h bis 25.000 kg/h sehr hoch. Da zeitliche Schwankungen in der Gemischzusammensetzung zwangsläufig signifikante Rückwirkungen auf die Eigenschaften der entstehenden Endprodukte haben, ist der Gemischaufbereiter bestrebt, fortlaufend Informationen über die momentane prozentuale Zusammensetzung des gerade entstehenden Stoffgemisches zu gewinnen. Aufgrund der Produzentenhaftung wird er sich auch um die Dokumentation derartiger Informationen bemühen.

Es entspricht dem derzeitigen Stand der Technik, in bestimmten Zeitintervallen aus dem Granulatstrom Proben zu ziehen und diese auf ihre gewichtsprozentuale Zusammensetzung hin off-line, d.h. prozeßfern im Prüflabor zu untersuchen. Hierbei wird die Gemischdichte mit Hilfe eines Dichtegradientenrohres bestimmt, aus der die Gewichtsanteile der Einzelkomponenten berechnet werden können, sofern die Stoffdichten der Mischungsanteile bekannt sind. Im Fall mineralisch gefüllter oder verstärkter Polymere, wie bei Pigmentkonzentraten, welche anorganische Pigmente enthalten, läßt sich die gewichtsanteilmäßige Gemischzusammensetzung auch durch Veraschen der Proben bestimmen. Eine weitere Möglichkeit, die Zusammensetzung solcher Gemische zu analysieren, besteht in der Anwendung der Fourier-Transform-Infrarot-Spektroskopie (FTIR). Diese Analysemethode macht allerdings eine langwierige Probenaufbereitung notwendig und erfordert einen sehr hohen gerätetechnischen Aufwand. Allen genannten Verfahren zur Ermittlung der gewichtsanteiligen Zusammensetzung solcher Polymer/Zuschlagstoff-Gemische ist gemeinsam, daß sie off-line durchgeführt werden, wodurch lange Totzeiten entstehen, während denen unter Umständen nicht typgerechte Ware compoundiert wird. Abgesehen davon, daß einige dieser Verfahren störanfällig sind (beispielsweise an den Proben haftende Luft bei Dichte-Messungen mittels Dichtegradientenrohr), liefern sie zudem keine Analysenergebnisse, welche Basis für die Entwicklung geschlossener Prozeßregelkreise sein können.

Zur möglichst prozeßzeitkonformen Ermittlung der gewichtsanteiligen Zusammensetzung der Gemische könnte prinzipiell auch ein on-line betreibbares, d.h. mit einem By-pass-Strom versorgtes Prozeßrheometer verwendet werden. Aus den damit kontinuierlich erfaßbaren rheologischen Stoffwertfunktionen kann zumindest im Prinzip auf eintretende Änderungen in der Gemischzusammensetzung geschlossen werden. Zu berücksichtigen bleibt allerdings, daß durch den Aufbereitungsprozeß bedingte Veränderungen polymerer Strukturparameter, welche unmittelbar auf die rheologischen Stoffeigenschaften zurückwirken, eine Veränderung in der Gemischzusammensetzung vortäuschen können. Diese Unsicherheit in der Interpretation der rheometrischen Ergebnisse sowie die unzulängliche, für die Prozeßführung noch keineswegs ausreichende Reduktion der Totzeit stehen dem Einsatz der on-line-Rheometrie zum Zwecke der Ermittlung von Gemischzusammensetzungen entgegen. Die on-line-FTIR Spektroskopie scheidet aufgrund des extrem hohen gerätetechnischen Aufwands sowie wegen der relativ langen Totzeiten aus dem Kreis jener Meßverfahren aus, mit denen die gewichtsanteilige Zusammensetzung von Polymer/Zuschlagstoff-Gemischen wirklich prozeßzeitkonform (= Real-time-Monitoring) zu ermitteln ist.

Bekannt ist ein Verfahren zur Messung des Dampfgehaltes stationärer und instationärer Zweiphasenströmungen mittels eines Gamma-Strahl-Dichtemeßsystems. Die Gamma-Strahlung wird beim Durchgang durch das Zweiphasengemisch um so weniger geschwächt, je größer der Dampfgehalt ist. Die verbleibende Reststrahlung wird in einem Szintillationsdetektorsystem registriert und in ein intensitätsproportionales Spannungssignal gewandelt. Das zu untersuchende Medium fließt durch ein dünnwandiges Rohr. Das Meßsystem ist indessen nicht geeignet für Polymerschmelzen, welche bei hoher Temperatur unter hohem Druck stehen.

Die Aufgabe bestand darin, ein Verfahren für die kontinuierliche Erfassung und Quantifizierung der gewichtsanteiligen Zusammensetzung von Polymer/Zuschlagstoff-Gemischen, die bei Aufbereitungsprozessen entstehen, zu finden. Dabei sollten die relevanten Rezepturdaten oder die entsprechenden Analogiegrößen in-line und damit weitgehend totzeitfrei mit hoher Präzision und guter Reproduzierbarkeit ermittelt werden können.

Es wurde gefunden, daß die Aufgabe gelöst werden kann mit einem Verfahren, welches auf dem Prinzip der radiometrischen Dicken- bzw. Dichtemessung aufbaut, und mit einer für die Ausübung dieses Verfahrens passenden Vorrichtung.

Die Erfindung betrifft somit ein Verfahren zur prozeßzeitkonformen Erfassung von Mischungsverhältnissen bei der Kunststoff- und Kautschukaufbereitung, dadurch gekennzeichnet, daß man mittels einer Vorrichtung bestehend aus einem Zylinderschuß (1) mit einem axialen Kanal (4) mit rechteckigem Querschnitt, mit Gleitflächen (5) und (6) für einen beiderseits des Zylinderschusses (1) quer zur Achse des Zylinderschusses (1) beweglichen Schlitten, welcher einerseits Halterungen (8) für eine Strahlenquelle und andererseits ein Szintillationsdetektorsystem (9) trägt, wobei die schmalen Seiten des Kanals (4) den Gleitflächen (5) und (6) zugewandt sind und die Wandstärke des Zylinderschusses (1) im Bereich der Verbindungsachse Strahlenquelle-Detektorsystem verringert ist, die durch den Kanal (4) strömende Polymerschmelze mit dem von der Strahlenquelle ausgehenden Meßstrahl durchstrahlt und aus der Abschwächung der Strahlungsintensität nach der Formel

$$k_F = \frac{1}{[\rho_F \cdot \mu_F - \rho_P \cdot \mu_P]} \cdot \left[ \frac{1}{L} \cdot \ln\left(\frac{I_{St}}{St,G}\right) - \rho_P \cdot \mu_P \right]$$

worin
$k_F$ = prozentualer Volumenanteil des Füllstoffes, Verstärkungsstoffes, Pigments oder der Blendkomponente,
$I_{St}$ = gemessene Strahlungsintensität am leeren Kanal (4),
$I_{St,G}$ = gemessene Strahlungsintensität am mit Schmelze gefüllten Kanal (4),
$\mu_P$ = Massenschwächungskoeffizient des Polymers,
$\mu_F$ = Massenschwächungskoeffizient des Füllstoffes, Verstärkungsstoffes, Pigments oder der Blendkomponente
$L$ = durchstrahlte Materiallänge,
$\rho_F$ = Dichte des Füllstoffes, Verstärkungsstoffes, Pigments oder Blendkomponente und
$\rho_P$ = Dichte des Polymeren bei der Schmelzentemperatur und dem Druck $\bar{p}$
bedeuten, den Volumenanteil $k_F$ ermittelt und daraus nach den Gleichungen
$k_P = 1 - k_F$,

$$a = \frac{k_P \cdot \rho_P}{[k_P(\rho_F - \rho_P) - \rho_F]}$$
$$b = 1 - a$$

und $b = 1 - a$
worin $k_P$ für den prozentualen Volumenanteil des Polymers und a für den prozentualen Gewichtsanteil des Polymers stehen, den prozentualen Gewichtsanteil b des Füllstoffes, Verstärkungsstoffes, Pigments oder der Blendkomponente errechnet.

Die Erfindung betrifft weiterhin eine Vorrichtung, bestehend aus einem Zylinderschuß (1) mit einem axialen Kanal (4) mit rechteckigem Querschnitt, mit Gleitflächen (5) und (6) für einen beiderseits des Zylinderschusses (1) quer zur Achse des Zylinderschusses (1) beweglichen Schlitten, welcher einerseits Halterungen (8) für eine Strahlenquelle und andererseits ein Szintillationsdetektorsystem (9) trägt, wobei die

4

schmalen Seiten des Kanals (4) den Gleitflächen (5) und (6) zugewandt sind und die Wandstärke des Zylinderschusses (1) im Bereich der Verbindungsachse Strahlenquelle-Detektorsystem verringert ist.

Beim erfindungsgemäßen Verfahren durchstrahlt ein von einer Strahlenquelle, beispielsweise Gamma- oder Röntgenquelle, ausgehender gebündelter Meßstrahl den gesamten durch einen Meßkanal strömenden Gemischstrom und die Meßkanalwandungen, wobei er beim Durchqueren dieser Materialschichten zum Teil absorbiert und partiell gestreut wird.

Die Abschwächung des Strahls hängt von der Dicke und der Dichte der penetrierten Materialschichten ab. Die auf die Wanddicken des Werkzeugkanals zurückzuführende Strahlungsabsorption läßt sich in einem sogenannten Leerrohrversuch, bei dem der ungefüllte Meßkanal durchstrahlt wird, ermitteln. Hält man die Meßkanalabmessungen und damit die Gemischschichtdicke konstant, so kann durch Messen der Intensität der den gefüllten Meßkanal durchdringenden Strahlung mit Hilfe einer hochstabilen Röntgenmeßkette unmittelbar die Gemischdichte quantifiziert werden. Für die Ermittlung der gewichtsanteiligen Zusammensetzung von Polymer/Zuschlagstoff-Gemischen werden noch zusätzlich weitere physikalische Zustandgrößen gemessen, nämlich an der Durchstrahlungsstelle mit Hilfe eines Präzisionsdruckaufnehmers der lokale Massedruck $\bar{p}$ und mittels eines in den Plastifikatstrom eintauchenden Thermoelements die repräsentative Gemischtemperatur $\bar{T}$. Unter Einbeziehung von thermischen Zustandsgleichungen, welche ganz allgemein die Abhängigkeit der Stoffdichte eines Mediums von Druck und Temperatur formulieren, können in Verbindung mit den Meßwerten $\bar{p}$ und $\bar{T}$ zu jeder Zeit die Stoffdichten der Einzelkomponenten bestimmt werden. Solche thermische Zustandsgleichungen enthalten stoffcharakteristische Materialparameter, welche bei der Aufnahme sogenannter p,v,T-Diagramme bestimmt werden und als Vorinformation in das erfindungsgemäße Meß- und Auswerteverfahren eingebracht werden.

Sind auf diese Art und Weise die momentanen, an der Durchstrahlungsstelle vorliegenden Dichtewerte der einzelnen Gemischkomponenten bekannt, so können unter Einbeziehung der mit der Röntgenmeßkette gemessenen und durch die Penetration des Gemischstromes reduzierten Strahlungsintensität unter Berücksichtigung von stoff- und strahlerabhängigen Massenschwächungskoeffizienten sowie von der durchstrahlten Gemischschichtdicke die Volumenanteile der einzelnen Gemischkomponenten am Gesamtvolumenstrom sofort parallel zum Prozeß bestimmt werden. Diese Volumenanteile sind leicht in die gesuchten Gewichtsanteile der Polymer/Zuschlagstoff-Gemische umzurechnen.

Die Abnahme der Strahlungsintensität dI beim Durchstrahlen einer Materieschicht ist proportional der aktuellen Intensität I, der Materiedichte $\rho$, dem Massenschwächungskoeffizienten $\mu$ sowie der Schichtdicke dx:

$$dI = -I \cdot \mu \cdot \rho \cdot dx . \quad (1)$$

Durch Integration erhält man

$$I = I_0 \cdot \exp(-\mu \cdot \rho \cdot L), \quad (2)$$

worin $I_0$ für die ungeschwächte Ausgangsintensität des Strahles und L für die gesamte durchstrahlte Meßlänge steht. Für den mit Schmelze gefüllten Strömungskanal ergibt sich die meßbare Strahlungsintensität zu

$$I_{St,P} = I_0 \cdot \exp[-(\mu_{St} \cdot \rho_{St} \cdot L_{St} + \mu_P \cdot \rho_P \cdot L_P)] \quad (3)$$

Wird der leere Strömungskanal durchstrahlt, so mißt man

$$I_{St} = I_0 \cdot \exp(-\mu_{St} \cdot \rho_{St} \cdot L_{St}) . \quad (4)$$

Aus den Gln. (3) und (4) ergibt sich

$$I_{St,P} = I_{St} \cdot \exp(-\mu_P \cdot \rho_P \cdot L_P), \quad (5)$$

$$\ln \left[ \frac{I_{St}}{I_{St,P}} \right] = \mu_P \cdot \rho_P \cdot L_P \quad (6)$$

Diese Gleichung besagt, daß die am Leerkanal ermittelte Strahlungsintensität $I_{St}$ eine Schwächung erfährt, die unmittelbar durch den Massenschwächungskoeffizienten des Polymers $\mu_P$, die Polymerdichte $\rho_P$ und die Kanalbreite $L_P$ festgelegt wird.

Der Massenschwächungskoeffizient des Kunststoffs läßt sich dadurch bestimmen, daß man bei p = 1 bar und definierter Plastifikattemperatur $I_{St,P}$ mißt und $\rho_P$ für die gewählten Zustandgrößen p,T aus Tabellenwerken entnimmt.

Für das erfindungsgemäße Verfahren ist die Gl. (5) wie folgt zu modifizieren:

$$I_{St,G} = I_{St} \cdot \exp[-(\mu_P \cdot \rho_P \cdot L_P + \mu_F \cdot \rho_F \cdot L_F)] \quad (7)$$

Der Index F steht darin für Füllstoff, Verstärkungsstoff, Pigment oder Blendkomponente, der Index P für Polymer, St für Stahl und G für Gemisch. Die fiktiven Meßlängen $L_F$ und $L_P$ müssen aus den volumentrischen Gemischanteilen bestimmt werden. Sind a die prozentualen Gewichtsteile Polymer und b die prozentualen Gewichtsteile Füllstoff, so ergibt sich aus einer Massen- und Volumenbilanz die Gleichung

$$a \cdot \frac{\rho_G}{\rho_P} + b \cdot \frac{\rho_G}{\rho_F} = 1 \qquad (8) \quad ,$$

worin die linksseitigen Terme für die Volumenprozente von Polymer und Füllstoff stehen:

$$k_P = a \cdot \frac{\rho_G}{\rho_P} \quad (9a) \qquad k_F = b \cdot \frac{\rho_G}{\rho_F} \quad (9b)$$

$$L_P = k_P \cdot L \quad (9c)$$
$$L_F = k_F \cdot L \quad (9d)$$

Die Dichte des Gemisches $\rho_G$ ergibt sich zu

$$\rho_G = \frac{\rho_P \cdot \rho_F}{[\rho_F \cdot a + \rho_P \cdot b]} \qquad \qquad (10)$$

Die Abhängigkeit der Polymerdichte $\rho_P$ vom Druck $\overline{p}$ und Temperatur $\overline{T}$ läßt sich sofort mit Hilfe einer thermischen Zustandsgleichung formulieren und berechnen. Unter Heranziehen dieser thermischen Zustandsgleichung und der Gleichung (10) lassen sich mit Hilfe der Beziehungen

$$k_F = \frac{1}{[\rho_F \cdot \mu_F - \rho_P \cdot \mu_P]} \cdot \left[ \frac{1}{L} \cdot \ln \left( \frac{I_{St}}{I_{St,D}} \right) - \rho_P \cdot \mu_P \right] ,$$

$$k_P = 1 - k_F ,$$

$$a = \frac{k_P \cdot \rho_P}{[k_P(\rho_F - \rho_P) - \rho_F]} \qquad und$$

$$b = 1 - a$$

die effektiven Gewichtsanteile von Polmyer (a) und Füllstoff Verstärkungsstoff, Pigment oder Blendkomponente (b) bestimmen.

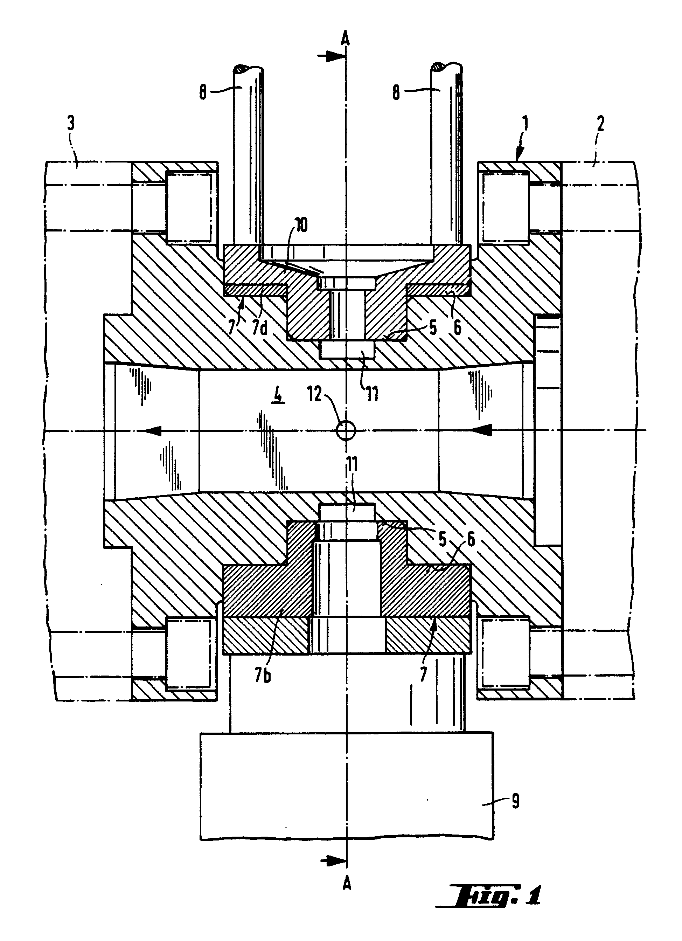

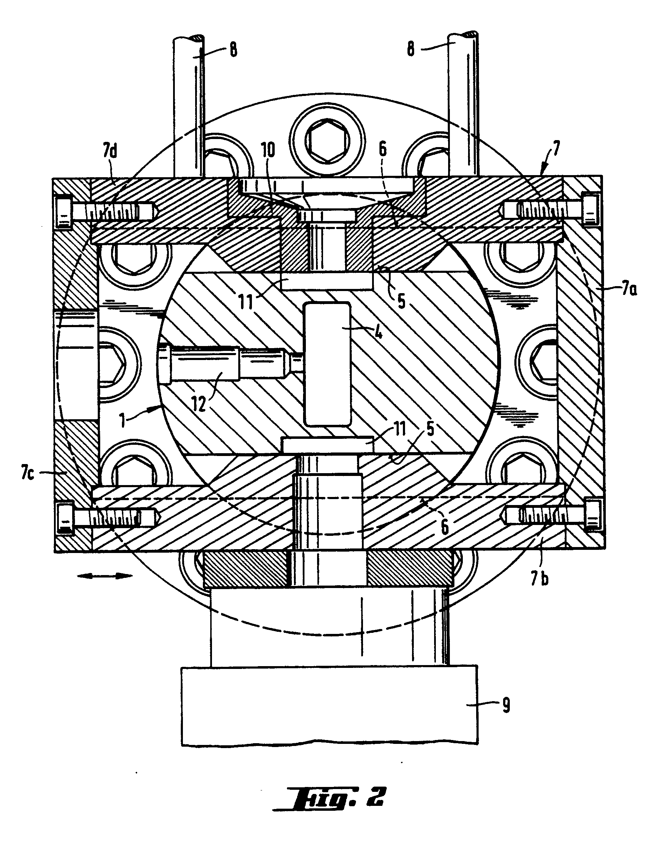

Die erfindungsgemäße Vorrichtung ist in den Figuren 1 und 2 dargestellt. Figur 1 zeigt einen Längsschnitt entlang der Hauptachse, d.h. entlang der Extrusionsrichtung, und Figur 2 zeigt einen senkrechten Querschnitt A-A in der Meßebene.

Gemäß Figur 1 besteht die erfindungsgemäße Vorrichtung aus einem Zylinderschuß (1), welcher zwischen Extruder (2) (angedeutet) und Extrusionswerkzeug (3) (angedeutet) montiert wird. Durch den Zylinderschuß (1) führt axial ein Kanal (4) durch welchen die Polymerschmelze vom Extruder (2) zum Extrusionswerkzeug (3) fließen kann. An zwei gegenüberliegenden Seiten ist der Zylinderschuß (1) abgefräst und bildet die Gleitflächen (5) und (6) für den Schlitten (7). Dieser Schlitten (7) trägt einerseits die Halter (8) für die Strahlenquelle (nicht gezeigt) und andererseits ein Szintillationsdetektorsystem (9). Zur Bündelung des Meßstrahls und zur axialen Strahlungsabschirmung ist im Schlitten (7) auf der Seite der Strahlenquelle eine Bleiabschirmung (10) angebracht. Weiterhin ist in der Meßebene A-A beiderseits des Zylinderschusses (1) jeweils die Wandstärke durch Ausfräsungen (11) verringert.

Figur 2 zeigt weitere Einzelheiten. Der Zylinderschuß (1) ist auf zwei Seiten planparallel abgefräst und bildet die Gleitflächen (5) und (6) (letztere nicht sichtbar) für den Schlitten (7), welcher der leichteren Demontage wegen aus vier Teilen (7a), (7b), (7c) und (7d) besteht. Der Kanal (4) besitzt vorzugsweise einen rechteckigen Querschnitt, wobei die schmalen Seiten des Kanals (4) den Gleitflächen (5) und (6) des Schlittens (7) zugewandt sind. Die Längsachse des Querschnitts ist somit identisch mit der Achse Strahlenquelle-Ausfräsungen (11) Szintillationsdetektor (9), welche senkrecht auf der Achse des Zylinderschusses (1) steht. In der Meßebene befindet sich eine Bohrung (12) für einen Massedruckaufnehmer sowie eine Bohrung (nicht gezeigt) für ein Thermoelement. Die Abmessungen des Kanals (4) sind derart gewählt, daß die durchstrahlte Schmelzeschicht dicker als 20 mm ist.

Der Schlitten mit der Strahlenquelle und dem Szintillationsdetektorsystem ist quer zur Achse des Zylinderschusses (1) beweglich, vorzugsweise vertikal. Die Bewegung kann von Hand über eine Schraubenspindel erfolgen, sie wird jedoch vorzugsweise durch einen Motorantrieb, beispielsweise mittels eines Schrittmotors, vorgenommen.

Ein jeweils mit dem Zylinderschluß (1) und dem Schlitten (7) verbundener Wegaufnehmer (nicht gezeigt) gibt kontinuierlich die momentane Schlittenposition an. Auf diese Weise läßt sich der gesamte Strömungskanal abtasten, wodurch zusätzliche Informationen über Verweilzeitspektren, Übergangsfunktionen und Geschwindigkeitsfelder beim Übergang von einer Gemischzusammensetzung zu einer anderen gewonnen werden. Das vorstehende Schlittenkonzept stellt nur eine von zahlreichen Ausführungsvarianten dar, die sich insbesondere für Laboruntersuchungen empfiehlt. Für den Produktionsbetrieb wird man hingegen eher eine vereinfachte Ausführung einsetzen, bei der Strahlenquelle und Detektorsystem relativ zum Strömungskanal (4) fest, d.h. nicht verschiebbar angeordnet sind.

Bei der in-line-Erfassung der gewichtsanteiligen Zusammensetzung von Polymer/Zuschlagstoff-Gemischen empfiehlt sich als Strahlenquelle eine Gamma- oder Röntgenquelle für kleine Prüfkammern mit relativ geringen Wanddicken und einem Plastifikatgemisch geringer Dichte oder eine Bremsstrahlungsquelle (Röntgenröhre oder Beschleuniger) für Meßvolumina großer Geometrie und/oder Medien hoher Dichte. Die Intensität der den Strömungskanal (4) durchdringenden Strahlung wird vorzugsweise mit einer hochstabilen Röntgenmeßkette ermittelt. Die Meßkette besteht aus einem Strahlungsdetektor und der zugehörigen Analogelektronik. Das Intensitätsmeßsignal wird ebenso wie das Druck- und Temperatursignal digitalisiert und einem Rechnersystem zur Meßdatenerfassung, Meßdatenauswertung, Zielgrößenbestimmung sowie zur Datendokumentation zugeführt. Im Zuge der Entwicklung von Prozeßführungskonzepten kann zudem noch ein digitaler Regler implementiert werden, der sich gleichfalls auf diese Daten abstützt. Für die Zielgrößenbestimmung (= Gewichtsanteile der Einzelkomponenten) werden noch stoffspezifische Parameter (Kohäsionsdruck, Kohäsionsvolumen, Molmasse eines Monomerbausteins) und Massenschwächungskoeffizienten für alle Einzelkomponenten benötigt, die vorab in den Rechner einzulesen sind.

Das erfindungsgemäße Verfahren und die erfindungsgemäße Vorrichtung lassen sich mit Vorteil für eine Reihe von Aufgaben verwenden:
- Kontinuierliche Messung der Dichte reiner Polymerer (Dichtekonstanz)
- Aufnahme von p,v,T-Diagrammen von Kunststoffen
- Ermittlung von Volumen- und Gewichtsanteilen bei Polymer/Füllstoff- bzw. Polymer/Verstärkungsmittel-Gemischen
- Ermittlung von Gewichtsanteilen Pigment bei der Masterbatch-Herstellung
- Ermittlung von Meßdaten für weiterführende Aussagen hinsichtlich mischtechnischer und thermischer Homogenität des Werkstoffs
- Sensor als Grundbaustein für einen geschlossenen Regelkreis zur Überwachung und Konstanthaltung von Füllstoffanteilen.

**Beispiel**

Auf einem gleichsinnig drehenden, dichtkämmenden Zweiwellenextruder wurden unter Variation des Granulierwerkzeugwiderstandes und damit des Massedrucks $\bar{p}$ in der Meßebene Polypropylen/Talkum-Gemische in unterschiedlicher prozentualer Zusammensetzung aufbereitet. Angestrebt wurden mit Hilfe einer volumetrischen Vordosierung der Einzelkomponenten Polypropylen und Talkum Gewichtsverhältnisse von 80:20, 70:30 und 60:40. Der effektive Talkumgehalt jeder Probe wurde zum einen off-line durch Veraschen, zum anderen in-line mit Hilfe des erfindungsgemäßen Verfahrens und der in den Figuren 1 und 2 gezeigten Vorrichtung bestimmt. Wie die Tabelle zeigt, stimmen die auf so unterschiedliche Weise ermittelten Talkumgehalte sehr gut überein.

Tabelle

| Gewichtsprozente | |
|---|---|
| gemessen in-line | ermittelt durch Veraschung |
| 20 | 20,60 |
| 30 | 29,96 |
| 37 | 37,80 |

**Ansprüche**

1. Verfahren zur prozeßzeitkonformen Erfassung von Mischungsverhältnissen bei der Kunststoff- und Kautschukaufbereitung, dadurch gekennzeichnet, daß man mittels einer Vorrichtung bestehend aus einem Zylinderschuß (1) mit einem axialen Kanal (4) mit rechteckigem Querschnitt, mit Gleitflächen (5) und (6) für einen beiderseits des Zylinderschusses (1) quer zur Achse des Zylinderschusses (1) beweglichen Schlitten, welcher einerseits Halterungen (8) für eine Strahlenquelle und andererseits ein Szintillationsdetektorsystem (9) trägt, wobei die schmalen Seiten des Kanals (4) den Gleitflächen (5) und (6) zugewandt sind und die Wandstärke des Zylinderschusses (1) im Bereich der Verbindungsachse Strahlenquelle-Detektorsystem verringert ist, die durch den Kanal (4) strömende Polymerschmelze mit dem von der Strahlenquelle ausgehenden Meßstrahl durchstrahlt und aus der Abschwächung der Strahlungsintensität nach der Formel

$$k_F = \frac{1}{[\rho_F \cdot \mu_F - \rho_P \cdot \mu_P]} \cdot \left[ \frac{1}{L} \cdot \ln\left(\frac{I_{St}}{I_{St,G}}\right) - \rho_P \cdot \mu_P \right]$$

worin
$k_F$ = prozentualer Volumenanteil des Füllstoffes, Verstärkungsstoffes, Pigments oder der Blendkomponente,
$I_{St}$ = gemessene Strahlungsintensität am leeren Kanal (4),
$I_{St,G}$ = gemessene Strahlungsintensität am mit Schmelze gefüllten Kanal (4),
$\mu_P$ = Massenschwächungskoeffizient des Polymers,
$\mu_F$ = Massenschwächungskoeffizient des Füllstoffes, Verstärkungsstoffes, Pigments oder der Blendkomponente
$L$ = durchstrahlte Materiallänge,
$\rho_F$ = Dichte des Füllstoffes, Verstärkungsstoffes, Pigments oder Blendkomponente und
$\rho_P$ = Dichte des Polymeren bei der Schmelzentemperatur und dem Druck p
bedeuten, den Volumenanteil $k_F$ ermittelt und daraus nach den Gleichungen
$k_P = 1 - k_F$ ,

$$a = \frac{k_P \cdot \rho_P}{[k_P(\rho_F - \rho_P) - \rho_F]}$$

und b = 1-a
worin $k_P$ für den prozentualen Volumenanteil des Polymers und a für den prozentualen Gewichtsanteil des Polymers stehen, den prozentualen Gewichtsanteil b des Füllstoffes, Verstärkungsstoffes, Pigments oder der Blendkomponente errechnet.
2. Vorrichtung, bestehend aus einem Zylinderschuß (1) mit einem axialen Kanal (4) mit rechteckigem Querschnitt, mit Gleitflächen (5) und (6) für einen beiderseits des Zylinderschusses (1) quer zur Achse des Zylinderschusses (1) beweglichen Schlitten, welcher einerseits Halterungen (8) für eine Strahlenquelle und andererseits ein Szintillationsdetektorsystem (9) trägt, wobei die schmalen Seiten des Kanals (4) den Gleitflächen (5) und (6) zugewandt sind und die Wandstärke des Zylinderschusses (1) im Bereich der Verbindungsachse Strahlenquelle-Detektorsystem verringert ist.

3. Verwendung der Vorrichtung gemäß Anspruch 2 zum Messen der Dichte einer Polymerschmelze.

**Fig. 1**

**Fig. 2**